# EUROPEAN PATENT APPLICATION

(11) **EP 3 078 356 A1**
(43) Date of publication of application: **12.10.2016**
(21) Application number: 14868438.4
(22) Date of filing: 28.07.2014
(51) Int. Cl.: A61F 5/56

(54) **DEVICE FOR PREVENTING SNORING**

(30) Priority: 04.12.2013 ES 201331768 P
(71) Applicant: Collantes Diaz, José María, 39420 Cantabria Bárcena Pie de Concha (Pujayo) (ES)
(72) Inventor: COLLANTES DIAZ, José María, 39420 Cantabria Bárcena Pie de Concha (Pujayo) (ES); VILLASANTE ARNAIZ, Oscar, 48003 Bizkaia Bilbao (ES)
(74) Representative: Villamor Muguerza, Jon
(86) International application number: PCT/ES2014/070610
(87) International publication number: WO 2015/082737

(57) **Abstract**

The invention relates to a device for keeping the mouth of a patient suffering from snoring problems closed or half-closed, said device being formed by two parts (1) and (2), the first part being fitted around the lips or mouth of the user by means of a strip of adhesive or glue, while the second part (2) is to be fixed, also by means of a strip of adhesive or glue, to the nose of the same user (4). The concentric area (5) of the first part (1) is free of adhesive or glue and is provided with openings (6) to allow the expulsion of air but not inhalation.

## Description

### TECHNICAL FIELD

The present invention relates to a device for preventing snoring, envisaged for being applied to the mouth and nose of the user, which partially prevents breathing through the mouth and allows breathing through the nose, which prevents classic snoring that some people suffer from while they sleep.

Therefore, the object of the invention is to prevent the massive entry of air through the mouth, which simultaneously makes it necessary to breath through the nose.

### PRIOR ART

As is known, one of the biggest problems today that can lead to serious problems when living together in a family unit is sleep apnea, or which is commonly understood as snoring.

Regardless of the fact that there are different degrees of snoring, ranging from mild snoring that barely bothers others living with the snorer, to very loud and unpleasant snoring which keeps the others living with the snorer awake, with the problems and drawbacks derived from this.

Snoring obviously occurs because the person who suffers from it sleeps with their mouth open, which leads to dry mouth and throat discomfort.

Taking into account that if one's mouth is closed or slightly half-open, snoring will not happen. Obviously any means preventing the mouth from opening will prevent the person with snoring problems from snoring, as they will become used to breathing through the nose

### DISCLOSURE OF THE INVENTION

The proposed device has been designed precisely to solve the drawbacks explained above because it is designed to keep the user's mouth closed, and the entry of air through the mouth is therefore prevented, forcing for the user to breathe through the nose, although air could also be expelled through the mouth, but at a minimum volume that will not lead to the classic snoring.

More specifically, the device of the invention is formed by means of a certain piece made of a flexible material having an oval-shaped part, provided with a perimetral strip with adhesive for being placed and glued around the mouth of the user, while through side bridges, the other part is arranged on the upper part of the nose, also being fixed to it by means of adhesive, leading to the mouth being kept closed and the nose unobstructed so that the user can breathe.

The part of the piece intended for being fixed around the mouth has a central part that is free of glue and is furthermore provided with openings to allow air to exit, but with a flow that will not lead to snoring.

It is therefore a device made of a flexible material and fitted around the mouth in the areas to be acted on, and which flexible material is, as stated, made up of two parts, a first part with glue that is adhered around the mouth, with a central part that is free of glue, that is located opposite the lips or mouth *per se,* and another second part that is adhered to the upper part of the nose, with an area also provided with glue for fixing it to said nose. The piece is empty between the part for being fixed to the nose and the part for being fixed to the mouth to allow the expulsion of air through the user's nostrils, thereby preventing snoring.

In addition to allowing the expulsion of air, the openings provided in the central part of the part that is adhered around the mouth enable being able to drink liquids or take medicines by means of a straw that goes through those openings.

The form or method of placing the device is essential, as will be explained in detail below.

The device is a solution to snoring, primarily restricting the loudest and most unpleasant snoring, which occurs by inhaling directly through the mouth, and snoring is the primary cause of problems and discomfort for other people living with a snorer.

Furthermore, the device of the invention improves the habit of breathing through the nose, which is beneficial compared with breathing through the mouth, so in addition to preventing snoring, the breathing process of the person using the device is improved.

Finally, the device improves other problems generated by breathing through the mouth when it is open in its entirety, which problems can correspond to dry mouth and primarily a dry throat, which is what causes the most discomfort to those suffering from snoring.

### DESCRIPTION OF THE DRAWINGS

To complement the description that will be made below and for the purpose of aiding to better understand the features of the invention, a set of drawings is attached to the present specification forming an integral part thereof, in which the following has been depicted in an illustrative and non-limiting manner:

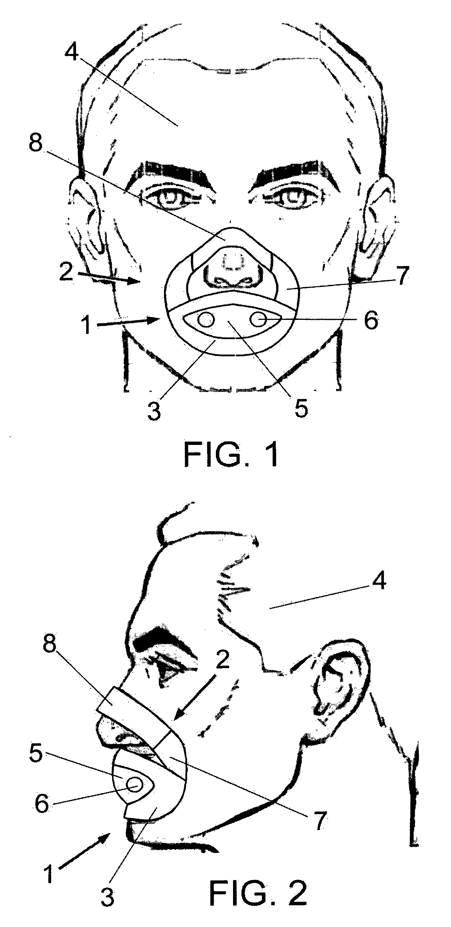

Figure 1 shows a practical application of the device according to a front view on a person's face.
Figure 2 shows a view of the same device in a lateral position applied on a person's face.
Figures 3 to 7 show five schematic views showing the method for placing the device.

### DETAILED DISCLOSURE OF THE INVENTION

As can be seen in the mentioned drawings, the device of the invention consists of two parts (1) and (2), said parts forming a one-piece body, such that the first part (1) has a considerably oval-shaped perimetral strip (3) provided with adhesive for being fixed around the lips or mouth of the user (4), while the central part or area (5) is free of glue or adhesive, being located in correspondence with the mouth, i.e., between the lips. Such area (5) is provided with a pair of openings (6) allowing the expulsion of air through them but not inhalation.

Both parts (1) and (2) are connected to one another by means of side bridges (7), with the special particularity that the second part (2) also has a rear strip of adhesive or glue for fixing it on the bridge or upper part of the nose (8) of the user (4), as depicted in the drawings.

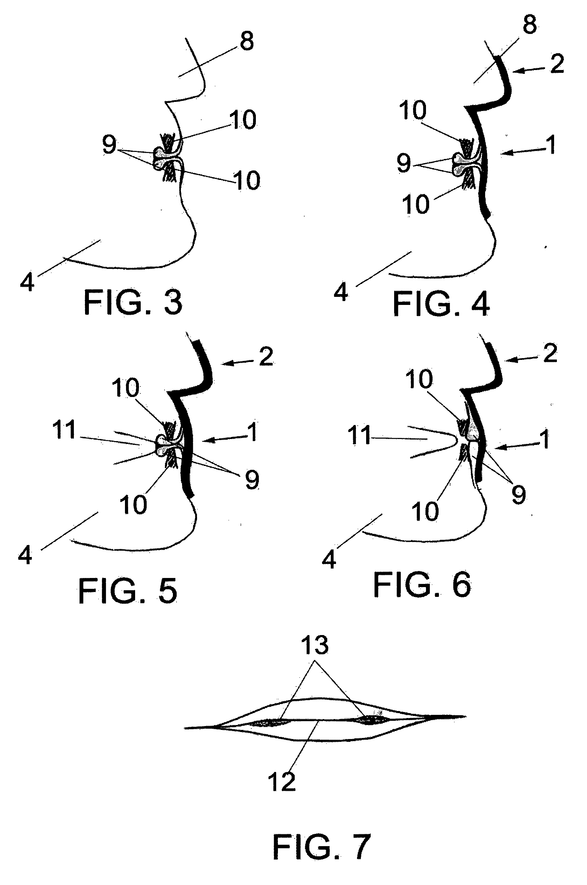

As explained, the device is placed on the face of the user (4) according to the following phases of the method:
1. The lips (9) are taken in and the teeth (10) are pressed against said lips (9), such that said lips (9) are between the teeth (10), as shown in Figure 3.
2. Then the device is fixed, as shown in Figure 4, performing said fixing by gluing with the adhesive provided on the rear face of parts (1) and (2).
3. Next the lips (9) are drawn against the outside of the teeth (10), using the tongue (11) to assist, as shown in Figure 5.
4. As a result of the preceding step, the lips (9) remain between the teeth (10) and the device, as shown in Figure 6.

The completed process results in a position of the mouth, teeth and lips with the device in place, preventing the inhalation of air due to the suction effect generated by the lips (9), while at the same time allowing the expulsion of air.

Finally and as can be seen in Figure 7, corresponding to a front view, the lips (9) are tightened in the central segment (12), while areas (13) through which the expulsion of air is allowed are determined at the end segments.

The device is made of a flexible material and is fitted around the face, in correspondence with the areas where it is applied with an adhesive material that neither damages nor irritates the skin.

## Claims

1. A device for preventing snoring, **characterized in that** it consists of two parts (1) and (2), the first part (1) being provided for being placed around the mouth of the user (4), while the second part (2) is provided for being placed on the nose (8) of the same user (4), with the special particularity that the first part (1) has a perimetral strip (3) with adhesive or glue for being fixed around the lips or mouth of the user (4), while the second part (2) has another strip of adhesive or glue for being fixed to the upper part of the nose (8).

2. The device for preventing snoring according to claim 1, **characterized in that** the central area (5) of the first part (1), located concentrically to the strip (3) with the adhesive, is free of adhesive or glue and is provided with a pair of openings (6) allowing the expulsion of air but not inhalation.

3. The device for preventing snoring according to the preceding claims, **characterized in that** parts (1) and (2) are connected to one another through side bridges (7).

4. The device for preventing snoring according to the preceding claims, **characterized in that** it is made of a flexible material and fitted around the face, in correspondence with the areas where it is applied with an adhesive material that neither damages nor irritates skin.
